**Europäisches Patentamt**

**European Patent Office**

**Office européen des brevets**

(19)

(11) Veröffentlichungsnummer: **0 036 552**
**B1**

# EUROPÄISCHE PATENTSCHRIFT

(12)

(45) Veröffentlichungstag der Patentschrift:
**19.09.84**

(51) Int. Cl.³: **C 07 D 323/06**, C 08 G 2/10

(21) Anmeldenummer: **81101741.7**

(22) Anmeldetag: **10.03.81**

(54) **Verfahren zur Reinigung von Trioxan.**

(30) Priorität: **25.03.80 DE 3011335**

(43) Veröffentlichungstag der Anmeldung:
**30.09.81 Patentblatt 81/39**

(45) Bekanntmachung des Hinweises auf die Patenterteilung:
**19.09.84 Patentblatt 84/38**

(84) Benannte Vertragsstaaten:
**BE DE FR GB IT NL**

(56) Entgegenhaltungen:
**AT - B - 255 131**
**DE - B - 1 493 688**
**DE - B - 1 543 528**

(73) Patentinhaber: **BASF Aktiengesellschaft,
Carl-Bosch-Strasse 38, D-6700 Ludwigshafen (DE)**

(72) Erfinder: **Rath, Hans Peter, Dr., Friedhofstrasse 7,
D-6718 Gruenstadt (DE)**

## Beschreibung

Trioxan wird in einem großtechnisch ausgeübten Verfahren durch sauer katalysierte Trimerisierung hergestellt. Dieses Verfahren ist in Ullmann's Encyklopädie der technischen Chemie, Band 11, Seite 699 (1976) ausführlich beschrieben. Danach wird die bei der Trimerisierung anfallende wäßrige Trioxanlösung mit einem organischen Lösungsmittel, vorzugsweise einem Halogenkohlenwasserstoff, extrahiert, das organische Lösungsmittel wird durch Destillation entfernt und das Rohtrioxan einer Reindestillation unterworfen.

Nach der FR-A-1 449 675 wird der Extraktion zur Entfernung der Verunreinigung und Nebenprodukte (Ameisensäure, Methylformiat, Formaldehyd) eine Wäsche des Extraktes mit wäßriger Natronlauge nachgeschaltet. Dabei geht ein Teil des wasserlöslichen Trioxans in die wäßrige Phase und muß daraus mühevoll wieder zurückgewonnen werden. Es hat sich ferner gezeigt, daß in vielen Fällen eine unerwünschte Reaktion des Alkalis mit dem organischen Lösungsmittel, insbesondere mit Halogenverbindungen, stattfindet. Dabei geht ein verhältnismäßig großer Teil des organischen Lösungsmittels, das gewöhnlich nach der Abtrennung vom Rohtrioxan gereinigt und zur Extraktion wieder verwendet wird, aus dem Kreislauf verloren und muß ersetzt werden. Außerdem bilden sich durch die erwähnte Reaktion unerwünschte Nebenprodukte.

Nach der DE-B-1 543 528 wird der Trioxan-Extrakt ebenfalls mit wäßrigem Alkali behandelt und das Extraktionsmittel abdestilliert, wobei vor, während oder nach der Destillation ein nicht flüchtiges, in Trioxan lösliches tertiäres Amin zugesetzt wird. Dieses dient zur Stabilisierung des Trioxans gegen Zersetzung zu Formaldehyd bzw. vorzeitige Polymerisation zu Polyoxymethylenen.

Der Erfindung lag nun die Aufgabe zugrunde, ein optimales Verfahren zur Reinigung von Rohtrioxan zu entwickeln. Insbesondere sollte ein Verlust an Trioxan, sowie die Bildung von Nebenprodukten bei der Alkali-Behandlung des Rohtrioxans vermieden werden. Ferner sollte die Reinheit des Trioxans und damit seine Polymerisationsfähigkeit gewährleistet werden. Es wurde nun gefunden, daß diese Aufgaben gelöst werden, wenn man bei dem bekannten Verfahren zur Herstellung von Trioxan das Rohtrioxan mit geschmolzenem Kaliumhydroxid oder Bariumhydroxid behandelt.

Gegenstand der Erfindung ist demzufolge ein Verfahren zur Reinigung von Trioxan, welches durch sauer katalysierte Trimerisierung von Formaldehyd, Extraktion des Trioxans aus der anfallenden wäßrigen Trioxanlösung mit einem Halogenkohlenwasserstoff, gegebenenfalls Waschen des Extrakts mit Wasser, und destillative Entfernung des Halogenkohlenwasserstoffes hergestellt wurde, durch alkalische Behandlung, bei der man das Rohtrioxan in der Schmelze bei einer mittleren Verweilzeit von 1 bis 60 min bei Temperaturen von maximal 160°C mit Kaliumhydroxid oberhalb von 97°C oder mit Bariumhydroxid oberhalb von 58°C behandelt und das gereinigte Trioxan destilliert.

Im Gegensatz zu dem Verfahren nach der FR-A-1 449 675 arbeitet man nicht mit einer wäßrigen Alkali-Lösung, sondern in der Schmelze. Dies ist deshalb möglich, weil überraschenderweise gefunden wurde, daß durch Trioxan (welches unter Normaldruck einen Siedepunkt von 114°C hat) der Schmelzpunkt von KOH bzw. Ba(OH)₂ von 410°C bzw. 408°C auf 97°C bzw. 58°C erniedrigt wird. Bei anderen Basen wird diese drastische Erniedrigung des Schmelzpunktes nicht beobachtet. Die alkalische Behandlung wird nicht vor, sondern nach der Abdestillation des organischen Lösungsmittels vorgenommen, so daß die unerwünschte Reaktion des Halogenkohlenwasserstoffs mit dem Alkali nicht stattfinden kann.

Bei dem erfindungsgemäßen Verfahren geht man aus von einem Troixan, das auf bekannte Weise durch sauer katalysierte Trimerisierung von Formaldehyd hergestellt wurde. Aus der dabei anfallenden wäßrigen Troixanlösung wird ein Gemisch aus Trioxan, Formaldehyd und Wasser abdestilliert. Aus diesem Gemisch wird das Trioxan mit einem Halogenkohlenwasserstoff, wie Methylenchlorid, Chloroform, Tetrachlorkohlenstoff oder insbesondere 1,2-Dichlorethan extrahiert. Der Halogenkohlenwasserstoff wird vor der Alkalibehandlung wieder abdestilliert.

Die bevorzugte Alkalibehandlung mit KOH wird so vorgenommen, daß man das auf Temperaturen von 97°C bis 115°C (bei Arbeiten unter Druck gegebenenfalls auch höher) erhitzte Trioxan diskontinuierlich oder bevorzugt kontinuierlich mit KOH bzw. mit einer Trioxan/KOH-Schmelze vermischt. Die Alkalibehandlung wird bei Temperaturen von maximal 160°C bei einer mittleren Verweilzeit von 1 bis 60, insbesondere 2 bis 20 min vorgenommen. Bei Verwendung von KOH wird bei Temperaturen oberhalb von 97°C, vorzugsweise oberhalb von 100°C gearbeitet, bei Ba(OH)₂ oberhalb von 58°C, vorzugsweise oberhalb von 70°C. Das gereinigte Trioxan wird abdestilliert und zweckmäßigerweise gleich anschließend einer Reindestillation unterworfen.

Das gereinigte Trioxan wird zur Herstellung von Oxymethylen-Polymeren durch kationische Polymerisation verwendet. Es hat sich gezeigt, daß dabei ein höhermolekulares Polyoxymethylen erhalten wird als mit nach FR-A-1 449 675 gereinigtem Trioxan. Dies läßt darauf schließen, daß die erfindungsgemäße Reinigung effektiver war.

Die in den Beispielen genannten Teile und Prozente beziehen sich auf das Gewicht.

## Beispiel 1

### (Vergleichsversuch)

In einem kontinuierlichen Verfahren werden aus einer 50%igen Formaldehydlösung (1,5 kg) in einem 2-l-Vierhalskolben mit Thermometer, Rührer, seitenstehender Kolonne und beheiztem Zulauf für Formaldehydlösung in Gegenwart von 300 g $H_2SO_4$ ein Gemisch aus Formaldehyd, Trioxan und Wasser abdestilliert, auf einer 2 m langen Füllkörperkolonne (NW 30, gefüllt mit V2A-Siebringen 5 mm) in ein trioxanreiches Gemisch und verdünnte Formaldehydlösung aufgetrennt. Der Kolonnenzulauf liegt bei 1,40 m Kolonnenhöhe.

Das trioxanreiche Gemisch wird dann im Gegenstrom mit 1,2-Dichlorethan extrahiert, wobei eine 50%ige Trioxanlösung entsteht. Zur Gegenstromextraktion wird eine Siebbodenkolonne mit 30 Böden, NW 50, verwendet.

Der Extrakt wird mit etwa 20 Vol.-% an destilliertem Wasser in einer Gegenstromwäsche behandelt. Daran schließt sich eine Destillation auf einer 2 m Füllkörperkolonne NW 30, gefüllt mit V2A Siebringen 5 mm an, bei der Dichlorethan quantitativ entfernt wird. Der Kolonnenzulauf befindet sich in 1,40 m Bauhöhe, das Rücklaufverhältnis beträgt 2 : 1 und der Trioxanabzug aus dem Sumpf beträgt 1 kg/h.

Das so erhaltene Trioxan wird in einem weiteren Schritt diskontinuierlich auf einer 2 m Füllkörperkolonne NW 30, gefüllt mit V2A-Siebringen 5 mm aufdestilliert. Dabei werden 10% Vorlauf bei einem Rücklaufverhältnis von 10 : 1 und weitere 80% als Hauptlauf bei einem Rücklaufverhältnis von 1 : 3 entnommen. Die Destillationsgeschwindigkeit beträgt 1,3 l/h. Der so erhaltene Hauptlauf wird in die Polymerisation eingesetzt. Dabei wird das 70°C heiße Trioxan mit 2,5% Dioxepan (Comonomeres) und 40 ppm Bortrifluori-di-n-butyl-ätherat (Katalysator) versetzt. Die Zugabe eines Reglers ist nicht erforderlich, da das Trioxan genügend regelnd wirkende Verunreinigungen enthält. Nach etwa 30 min ist die Polymerisation beendet. Der entstandene Polymerisationsblock wird zermahlen, das erhaltene Pulver nach Zusatz von Sodalösung (zum Desaktivieren des Katalysators), Antioydans und Wärmestabilisator bei 200°C extrudiert. Am extrudierten Produkt wurde ein Schmelzindex (nach DIN 53 735 bei 190° und 2,16 kp) von 7,1 gemessen, was auf eine schlechte Monomerenqualität hindeutet.

## Beispiel 2

### (nach der FR-A-1 449 675)

Es wird gearbeitet wie in Beispiel 1, wobei der Extrakt aber einer Alkaliwäsche unterworfen wird. Dazu werden 80 Teile Extrakt mit 20 Teilen 10%iger wäßriger NaOH vermischt und bei 40°C 15 min lang behandelt. Aus dem gewaschenen Extrakt wird wie in Beispiel 1 das Lösungsmittel abdestilliert, das Trioxan durch fraktionierte Destillation gereinigt und anschließend polymerisiert. Dabei werden 0,03% Methylal als Regler zugesetzt. Am extrudierten Polymerisat wird ein Schmelzindex von 4,2 gemessen.

## Beispiel 3

Es wird zunächst gearbeitet wie in Beispiel 1. Der Extrakt wird mit Wasser gewaschen und anschließend einer Destillation zur Entfernung des Dichlorethans unterworfen. 2 kg Rohtrioxan werden dann mit 5 g KOH vermischt und die Mischung 20 min lang unter Rühren am Rückfluß erhitzt. Dann wird auf 80° abgekühlt und das auskristallisierte KOH abfiltriert. Das so behandelte Trioxan wird anschließend durch fraktionierte Destillation gereinigt und nach Zusatz von 0,03% Methylal wie in Beispiel 1 polymerisiert. Am extrudierten Polymerisat wird ein Schmelzindex von 3,0 gemessen.

## Beispiel 4

Beispiel 3 wird wiederholt, wobei jedoch statt KOH $Ba(OH)_2$ eingesetzt wurde. Die Schmelzviskosität betrug 3,4.

## Patentansprüche

1. Verfahren zur Reinigung von Trioxan, welches durch sauer katalysierte Trimerisierung von Formaldehyd, Extraktion des Trioxans aus der anfallenden wäßrigen Trioxanlösung mit einem Halogenkohlenwasserstoff, gegebenenfalls Waschen des Extrakts mit Wasser, und destillative Entfernung des Halogenkohlenwasserstoffes hergestellt wurde, durch alkalische Behandlung, dadurch gekennzeichnet, daß man das Rohtrioxan in der Schmelze bei einer mittleren Verweilzeit von 1 bis 60 min bei Temperaturen von maximal 160°C mit Kaliumhydroxid oberhalb von 97°C oder mit Bariumhydroxid oberhalb von 58°C behandelt und das gereinigte Trioxan destilliert.

2. Verfahren nach Anspruch 1, dadurch gekennzeichnet, daß man die Alkalibehandlung mit KOH bei Temperaturen zwischen 100°C und 160°C durchführt.

3. Verwendung des nach Anspruch 1—3 gereinigten Trioxans zur Herstellung von Oxymethylen-Polymeren durch kationische Polymerisation.

## Claims

1. A process for the purification of trioxane, which has been prepared by acid-catalyzed trimerization of formaldehyde, extracting the trioxane from the resulting aqueous trioxane solution with a halohydrocarbon, optionally washing the extract with water, and removing the halohydrocarbon by distillation, by treatment with an alkali, wherein the crude trioxane is treated in the melt, the mean residence time being from 1 to 60 minutes and the maximum temperature being 160°C, with potassium hydroxide at above 97°C or with barium hydroxide at above 58°C, and the purified trioxane is distilled.

2. A process as claimed in claim 1, wherein treatment of the trioxane is carried out with KOH at a temperature of from 100 to 160°C.

3. The use of the trioxane purified according to claim 1 or 2 for the production of oxymethylene polymers by cationic polymerization.

**Revendications**

1. Procédé d'épuration, par traitement alcalin, de trioxanne qui a été obtenu par trimérisation catalysée acide de formaldéhyde, extraction du trioxanne de la solution de trioxanne aqueuse obtenue avec un hydrocarbure halogéné, éventuellement lavage de l'extrait avec de l'eau et élimination par distillation de l'hydrocarbure halogéné, caractérisé par le fait qu'on traite le trioxanne brut dans la masse fondue, avec une durée de séjour moyenne de 1 à 60 min, à des températures d'au maximum 160°C, avec de l'hydroxyde de potassium au-dessus de 97°C ou avec de l'hydroxyde de baryum au-dessus de 58°C, et l'on distille le trioxanne purifié.

2. Procédé selon la revendication 1, caractérisé par le fait qu'on effectue le traitement alcalin avec du KOH à des températures comprises entre 100°C et 160°C.

3. Utilisation du trioxanne purifié selon les revendications 1 à 3 pour la préparation de polymères d'oxyméthylène par polymérisation cationique.